(19) Europäisches Patentamt
European Patent Office
Office européen des brevets

(11) Publication number: **0 019 345**
**B1**

(12) **EUROPEAN PATENT SPECIFICATION**

(45) Date of publication of patent specification: **19.06.85**

(51) Int. Cl.[4]: **C 07 D 501/06** // C07C101/20

(21) Application number: **80200702.1**

(22) Date of filing: **11.08.78**

(60) Publication number of the earlier application in accordance with Art. 76 EPC: **0 001 133**

(54) **Process for the preparation of a (D-alpha-amino-(p-hydroxyphenyl)-acetamido) group containing cephalosporanic acid derivatives.**

(30) Priority: **06.09.77 NL 7709812**

(43) Date of publication of application:
**26.11.80 Bulletin 80/24**

(45) Publication of the grant of the patent:
**19.06.85 Bulletin 85/25**

(84) Designated Contracting States:
**BE CH DE FR GB LU NL SE**

(56) References cited:
**DE-A-2 418 719**
**GB-A-1 460 914**
**US-A-3 946 003**

**The file contains technical information submitted after the application was filed and not included in this specification**

(73) Proprietor: **Gist - Brocades N.V.**
**Wateringseweg 1 P.O. Box 1**
**NL-2600 MA Delft (NL)**

(72) Inventor: **Henniger, Peter Wolfgang**
**Lammenschansweg 65**
**NL-2313 DK Leiden (NL)**
Inventor: **Van Der Drift, Johannes Karel**
**Marterstraat 39**
**NL-2623 ED Delft (NL)**
Inventor: **Van Veen, Gerard Joannes**
**Thorbeckelaan 102**
**NL-2641 XJ Pijnacker (NL)**
Inventor: **Kapur, Jagdish Chander**
**Roland Holstlaan 799**
**NL-2624 KB Delft (NL)**

(74) Representative: **Huygens, Arthur Victor, Dr. et al**
**c/o Gist-Brocades N.V. Patent & Trademarks Department Wateringseweg 1 PO-Box 1**
**NL-2600 MA Delft (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 019 345 B1

**Description**

The invention relates to a process for the preparation of a [D-α-amino-(p-hydroxyphenyl) acetamido] group containing cephalosporanic acid derivatives, and more specifically of; 7-[D-α-amino-(p-hydroxyphenyl)acetamido]-3-methyl-3-cephem-4-carboxylic acid (commonly named cefadroxil), 7-[D-α-amino-(p-hydroxyphenyl)acetamido]-3-acetoxymethyl-3-cephem-4-carboxylic acid, 7-[D-α-amino-(p-hydroxyphenyl)acetamido]-3-[(1H)-1,2,3-triazol-5-yl-thiomethyl]-3-cephem-4-carboxylic acid (commonly named cefatrizine) and similar cephalosporanic acid derivatives, with — instead of the (1H)-1,2,3-triazol-5-yl-group — other heterocyclic residues, which may be optionally substituted by a lower akyl group, and non-toxic, pharmaceutically acceptable salts thereof.

The invention relates to a process for the preparation of cephalosporanic acid derivatives containing a [D-α-amino-(p-hydroxyphenyl)acetamido] group, by the acylation of derivatives of 7-aminocephalosporanic acid, wherein the 3-acetoxymethyl substituent has been optionally replaced by other substituents, with an acylating agent derived from p-hydroxyphenylglycine. More specifically the invention relates to a process for the preparation of cefadroxil, cefatrizine and similar derivatives by the acylation of a compound having the following formula

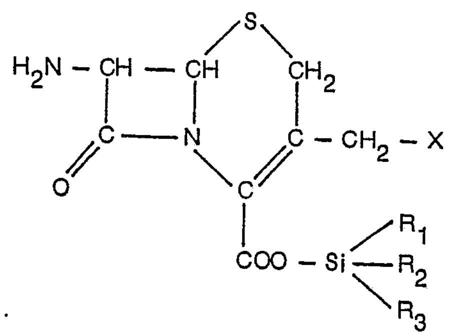

(I)

and/or

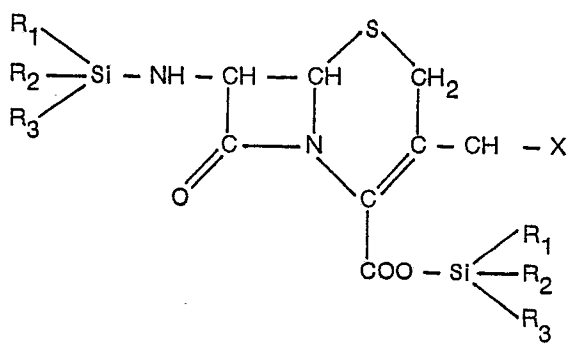

(II)

wherein $R_1$, $R_2$ and $R_3$ represent the same or different alkyl groups of 1—3 carbon atoms, and each preferably a methyl group, and wherein X represents a residue selected from the group consisting of hydrogen, acetoxy or a five membered heterocyclic ring, containing one or more heteroatoms selected from nitrogen, oxygen and/or sulphur and optionally substituted by $C_1$—$C_3$ alkyl, this ring being attached to the 3—$CH_2$ group via a sulphur atom, and wherein an endocyclic —NH-radical, if present, has optionally been silylated, with an acylating agent according to the general formula

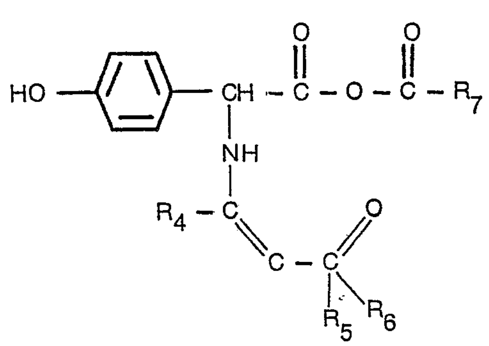

(III)

wherein

$R_4$ represents alkyl containing 1—3 carbon atoms and preferably methyl,

$R_5$ represents hydrogen or alkyl containing 1—3 carbon atoms and preferably methyl,

$R_6$ represents lower alkoxy containing 1—3 carbon atoms and preferably methoxy and

$R_7$ represents lower alkoxy containing 1—2 carbon atoms and preferably methoxy.

A process for the preparation of 6-[D-α-amino-p-(hydroxyphenyl) acetamido]- penicillanic acid is generally known from e.g. British Patent Specification no. 1,339,605 disclosing indeed the preparation of this compound according to Examples 1—5, but in economically non-interesting yields (Example 1: 43% of a purity of 80%; Example 3: 20% of a purity of 16%; Example 4: 37% of a purity of 93%; while the final parts of Examples 2 and 5 only indicate the presence of the desired compound amoxycillin.

On the other hand, a process which comprises the acylation of a previously silylated cephalosporanic acid derivative according to the formula I and/or II with a mixed anhydride according to the general formula III, is generally known from e.g. the published Dutch application NL 6912811, the British Patent Specification no. 1073530 and more particularly from the British Patent Specification no. 1460914.

However, especially from e.g. Examples 3 and 13 of the latter specifications, a person skilled in the art cannot derive with sufficient clarity, that an acylation of e.g. 7-amino-3-((1H)-1,2,3-triazol-5-yl)-thiomethyl-3-cephem-4-carboxylic acid, or its trimethylsilyl derivative, by means of e.g. ethoxycarbonyl D-α-(1-carbomethoxy-propen-2-yl)amino-p-hydroxyphenyl acetate, could be carried out with economically attractive yields.

More particulaly in Example 13, page 21, the reaction of 0.02 mol of 7-amino-3-1,2,3-triazol-5-yl)-thiomethyl-3-cephem-4-carboxylic acid, which has previously been silylated with 0.06 mol trimethylchlorosilane in the presence of 0.06 mol of about 2:1 mixture of triethylamine and N,N-dimethylaniline, with a reaction mixture prepared in the usual way in acetonitrile from 0.05 mol of the Dane salt and ethylchloroformate, seems to lead only to rather unattractive yields (see e.g. page 21 line 38 in connection with lines 5—10 of the same page).

Also other patent literature relating to the preparation of this type of cephalosporanic acid derivative showed only economically unattractive processes, e.g. US patent B 516.047, Example 1, column 7, line 44, Example 5A, line 52 and Example 6, US patent 3,946,003, Example 1 (column 11, line 65) and Example 3 (column 12, line 62).

Moreover, according to column 4, lines 1—5 and Example 1 (column 10), it seems necessary to silylate the phenolic p-hydroxy group first in order to obtain practical results, which necessity, moreover, also appears from Example 1 on pages 13 and 14 of the German application DT—2600880 and Examples 1—5 of German application DT—2626280.

Therefore, it was less attractive for people skilled in the art to develop the method described in e.g. the British specification 1.339.605 or similar methods for the preparation of cephalosporanic acids carrying the same p-hydroxy-phenylglycyl residue, than to look for alternative acylation methods in the search for a new and improved method for the preparation of the desired compounds with an economically attractive yield and a relatively high purity. An additional modern requirement for a process to be used on a technical scale, is, moreover, that inevitably occuring impurities can be removed in a cheap and simple manner so as to achieve the conditions of purity dictated by health authorities.

It is known from literature to prepare penicillanic acid and cephalosporanic acid derivatives by acylating 6—APA or 7-A(D)CA and their derivatives with the hydrochloride of 2-phenylglycinechloride and derivatives thereof having a substituted phenyl group, whereby the said acid chloride is obtained by reacting the substituted phenylglycine with reagents like phosphorus pentachloride, thionyl chloride and phosgene. Although improved processes for the preparation of D-(—)-2-(p-hydroxyphenyl)glycylchloride-hydrochloride and the crystalline hemidioxane solvate thereof are known from British Patent Specifications no. 1460915 and no. 1466637, the acylation of 6—APA or 7—A(D)CA or its 3-methyl modifications with the above mentioned acylating agent did not lead to the desired result, mainly because either the product formed was too impure to make its purification to the required quality worthwhile, or the starting D-2-(p-hydroxyphenyl)glycyl chloridehydrochloride of the required quality (purity) is only available at economically unattractive prices, if at all.

The occurring impurities found, if an acid chloridehydrochloride in economically necessary amounts for acceptable prices is used, appear to be in agreement with the indications about the accompanying impurities in the final products and the low yields of the rather similar acylation with the said D-(—)-2-(p-hydroxyphenyl)glycyl chloridehydrochloride of 7-amino-cephalosporanic acid derivatives according to British Patent Specification no. 1460916 (viz. Examples 2, 4 and 9, especially page 17, lines 32—34 and Example 8 referring to further purification of the desired product) and British Patent Specification no. 1460914 (viz. Examples 2, 5, 9 and 10). In German Application DT 2520647 page 2, lines 1—9 is also disclosed in this connection, that generally used acylating agents such as acid halides cannot be properly applied in the amoxicillin synthesis. It is true that a process for the preparation of amoxicillin trihydrate was also known from the published German patent application DT 2611286, comprising the acylation of previously silylated 6—APA, with D-(—)-2-p-hydroxyphenylglycylchloride hydrochloride and leading to yields which seem to approach to some extent at least present practical requirements.

However, for the preparation of this starting material according to e.g. British patent specifications nos. 1460915 and 1466637 phosgene is applied in a process relatively difficult to manage, in which a solid is

reacted with a gas. Such an application is extremely expensive in a large number of countries with very stringent safety regulations, if indeed it may be applied at all.

For the same reason the process described in British patent specifications nos. 1268536 and 1341827, disclosing the preparation of 6-isocyanatopenicillanic acid and 7-isocyanatocephalosporanic acid derivatives from 6—APA and 7—A(D)CA esters with phosgene and its subsequent reaction to form penicillins or cephalosporins, is left out of consideration for the preparation of amoxicillin and of cephalosporanic acid derivatives having the corresponding side chain in 7-position.

On the other hand, German Patent Application P 2520647, for example, discloses a process for the preparation of α-amino-acylpenicillanic acid derivatives and — inter alia — amoxicillin, in which

(i) 6—APA is contacted with an excess of a strong tertiary amine base (e.g. triethylamine) in an inert, water-insoluble, organic solvent (e.g. methylene chloride or chloroform), resulting in a solution of a salt of 6—APA with the base in said solvent,

(ii) the remaining strong tertiary amine base is neutralised in the solvent, e.g. by additon of N,N-dimethylacetamide hydrochloride,

(iii) the obtained neutralised solution is contacted with a solution of a mixed anhydride of a short chain alkoxyformic acid and a N-protected derivative of D-2-amino-p-hydroxyphenyl acetic acid, in which the N-protecting group is labile for acid, in a water-insoluble, inert, organic solvent at a temperature of −50° to +30°C, preferably −30° to 0°C, resulting in a solution of a N-protected amoxicillin derivative,

(iv) the solution so obtained is contacted with water and a strong acid (such as hydrochloric acid or p-toluene-sulphonic acid) at room temperature or cooled to, e.g. 0°C, in order to remove the acid-labile N-protecting group and

(v) the thus obtained amoxicillin is isolated from the aqueous system.

Less attractive features of this process are, inter alia, that the process is carried out at low concentrations, that solvents become mixed so that recovery thereof becomes more difficult and that, when adding the dimethylacetamide hydrochloride, due to local high concentrations, 6—APA, 7—ADCA or 7—ACA and its derivatives sometimes crystallizes, so that a very accurate dosing scheme is required.

Furthermore, a number of patent specifications disclose preparation methods of α-aminoacyl penicillanic acid derivatives by acylating 6—APA with mixed anhydrides derived from modified Dane salts of D-2-amino-(p-hydroxyphenyl) acetic acid, e.g. those described in German patent applications nos. 1302847, 2020133 and 2065879 and British Patent Specifications nos. 1327270 and 1347979.

However, the yields resulting from the use of such Dane salts appeared to be unsatisfactory as well for the purpose of the present invention, and moreover, these Dane salts appeared not to be commercially available.

Dutch patent application no. 6401841 further discloses the protection of the carboxylic group of 6—APA, 7—ACA and other amino acids by reacting it with dihalogensilane derivatives. These bi-functional silicon compounds are more easily accessible than the mono-functional trialkylhalogensilanes and the application thereof does lead to improved yields in a number of cases, as appears e.g. from British Patent Specification no. 1266544, disclosing the preparation of intermediate organosilane penicillins by reaction of 6—APA with the aid of said bi-functional silicon compounds. The organosilane derivatives are acylated to form, e.g. ampicillin. An expert, on account of the contents of this patent application would have expected that the application of the organosilane penicillins described therein, would lead to interesting yields in the preparations of amoxycillin too. However, very surprisingly this expectation could not be confirmed by the results of initial experiments.

From later patent specifications, e.g. British patent specification nos. 1356737, 1404846 and 1459999 it is known to employ trivalent phosphorous derivatives instead of the above-mentioned silicon derivatives. A disadvantage of these phosphorous derivatives is that they cost 10 to 20 times more than the said silicon derivatives and this in combination with the toxicity and spontaneous inflammability of the di(lower alkyl)phosphorus derivatives as indicated in Inorganic Synthesis 15 (1974) pages 191—3, makes the phosphorus compounds a poor substitute for the silicon compounds.

As a result of extensive research and development an improved process for the preparation of cephalosporanic acid derivatives containing a [D-α-amino-p-(hydroxyphenyl)acetamido] group has surprisingly been found. This process is characterized in that

a) a 7-aminocephalosporanic acid derivative or a 3-methyl modification thereof is reacted in a dry, inert, organic solvent with a trialkylsilyl group supplying agent in approximately 2 equivalents with respect to 7—ADCA or 7—ACA and approximately 3 equivalents with respect to those 3-methyl modifications which contain a —NH— radical in the residue being attached to the 3-methylene group, whereby the trialkylsilyl group supplying agent in the reaction mixture has been mutually balanced by adjusting at the end of the reaction to an empirically determined signal value as defined in the specification,

b) the obtained reaction mixture, containing a derivative of 7-aminocephalosporanic acid or 3-methyl modification thereof according to the formula

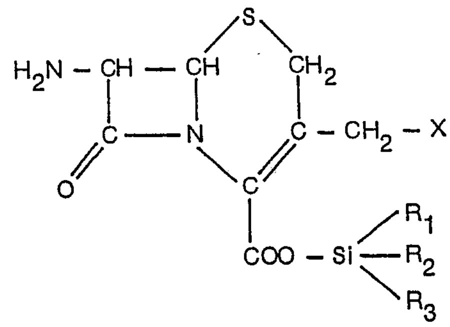

(I)

and/or

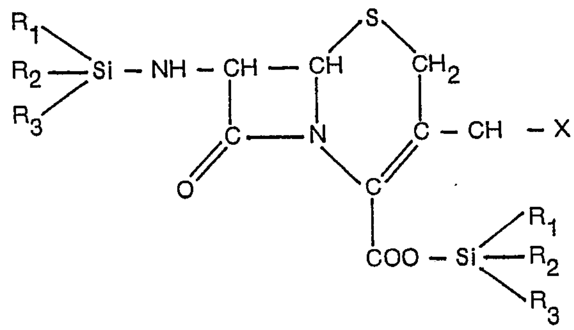

(II)

wherein each of $R_1$, $R_2$1and $R_3$ represents the same or different alkyl group of 1—3 carbon atoms and each preferably represents a methyl group and X represents a residue selected from the group consisting of hydrogen, acetoxy or a five-membered heterocyclic thio ring, containing one or more heteroatoms selected from nitrogen, oxygen and/or sulphur and optionally substituted by alkyl containing 1—3 carbon atoms, is cooled and subsequently is rapidly mixed under anhydrous conditions with a cooled solution of at least an equimolecular amount of a mixed anhydride according to formula (III), which solution has been prepared by reacting the corresponding Dane salt with an alkyl chloroformate under anhydrous conditions in a water immiscible, inert, organic main solvent to which 0—25% by volume of an inert cosolvent has been added and in the presence of a catalytic amount of a tertiary amine, whereafter

c) the acylation reaction is continued at a temperature below 0°C, followed by the recovery of the desired compound by methods known per se. The reaction is continued at a temperature of −10°C or lower and preferably from −20°C to −30°C during 0.25 to 3 hours and preferably one to 2.5 hours and the reaction mixture is poured out in water under exactly establishing the pH at a value below 2.5. Preferably the pH is established at a value between 0.8 and 1.2.

The dry, inert organic solvent for the silylation reaction is, preferably a water-immiscible solvent. The preferred dry, inert water-immiscible solvent is methylene chloride and the silylating agent is preferably trimethylchlorosilane (TMCS), in the presence of a tertiary amine. However, good results may also be obtained with trimethylsilylacetamide, bis(trimethylsilyl)acetamide, bis(trimethylsilyl)urea and hexamethyldisalazane.

The term "mutually balanced", as used under step (a) hereinbefore, can be clarified as indicated below.

It has been found, that optimal final yields can only be reached in a reproduceable manner by means of a very accurate balance between the amounts of agents employed to produce the silylated intermediate, especially in view of the desired optimal conversion with the prepared solution of the mixed Dane anhydride. It will be appreciated by persons skilled in the art, that — to point at just the main causes leading to undesirable deviation from the ideal balance — small errors in measuring out volumes of weighing out amounts are in practice inevitable, especially in experiments on very small scale (a few millimoles) or in production on large, industrial scale as well are impurities of technical reagents. What is needed then, is a "tool" whereby this necessary balance can be checked *in situ* and if necessary restored after completion of the silylation. The necessity and the advantage of "measuring this mutual balance" and its eventual restoration is a general feature of silylation procedures performed on 7—A(D)CA and the like, but is of preponderant importance when the silylation is carried out with the most economic agent combination, e.g. trimethylchlorosilane and triethyl amine, since such a combination — unlike single silylation agents like N,O-bistrimethylsilyl acetamide — does not provide for some sort of internal mutual balance by itself.

In the search for a suitable and at the same time simple method, by which this balance could be measured and if necessary corrected *in situ*, under technical conditions, it was surprisingly found, that for example the use of a good quality pH meter adequately fulfilled this demand. If for example a Radiometer pH meter type TTT2, C is employed in connection with a Radiometer GK 2401C electrode or an Ingold, so-called cold electrode at a temperature between 15 and 25°C, a pH scale value in the empirical range between 5.5 and 7.5 and preferably between 6.0 and 7.2 should prevail at the end of the silylation reaction. If this is not the case a small additional amount of trimethylchlorosilane or e.g. triethylamine is added in order to effect an improved mutual balance.

It has appeared to be irrelevant whether or not the above indicated optimal pH scale interval does in fact correspond to an actual pH interval of the same order (6.0—7.2) to be measured after dissolution of the silylation mixture in water, since scale interval readings on other suitable pH-meters can be gauged to the one obtained with the indicated pH meter type. A feature of the present invention therefore is recognition of the need to control the mutual balance of agents in the silylation reaction as well as prescription of a simple method to solve this fundamental problem *in situ.*

The silylation is preferably carried out in dry methylene chloride containing 2 or 3 equivalents of a tertiary amine, such as triethylamine, and an equivalent amount of TMCS (about 2 equivalents for e.g. cefadroxil and about 3 equivalents for e.g. cefatrizine).

The dry, water-insoluble main solvent used for the preparation of the so-called Dane anhydride may be dry methylene chloride to which dimethylformamide, sulfolane, tetrahydrofuran, N-methyl-pyrrolidone, 1,4-dioxane, acetonitrile, dimethylacetamide or tetramethylurea, or a mixture thereof, is added as a co-solvent to at most 25% by volume, or methylisobutylketone as main solvent to which one or more co-solvents mentioned above optionally may be added up to 25% by volume. Preferably potassium or sodium D-α-(1-carbomethoxy-propen-2-yl)-amino-p-hydroxy-phenyl-acetate is reacted with, preferably, methyl chloroformate in contrast with the opinions hitherto held for true, as may be evident from Houben-Weyl, Methoden der Organischen Chemie, 4th Edition (1974) Volume XV/2, Synthese von Peptiden, Part II, page, 172. N-methylmorpholine or N,N-dimethylbenzylamine is preferably used as a catalyst. The chloroformate is preferably added to the Dane salt, while the reaction is preferably carried out at a temperature of −10°C or lower, preferably at a temperature between −10°C and −35°C. Mixtures of methylene chloride and the indicated co-solvents, with to about 20% by volume and preferably up to 10% by volume of co-solvent in the starting mixture, are proposed as the optimal solvents for the preparation of the Dane mixed anhydride. Preferably, tetrahydrofuran, N,N-dimethylacetamide, N,N-dimethylformamide, N-methylpyrrolidone and N,N,N',N'-tetramethylurea are used as co-solvents.

According to a further preferred process the solution of the anhydride is cooled to a temperature of −15°C or lower and a cooled solution of silylated 7—ACA or 3-methyl modifications thereof, is added rapidly with efficient stirring so that a temperature of −15°C to −30°C is reached, whereafter the reaction mixture is stirred for a further 0.5 to 3 hours. A small excess of the Formula III compound is preferably employed, but the excess is dependent on the nature of the substituent at the 3-methyl group of the cephalosporanic nucleus.

For the preparation of e.g. cefatrizine, the initially prepared compound is preferably isolated from the reaction mixture in the form of the corresponding methanolate or propyleneglycolate. The solvates of cefatrizine may be converted into non-toxic, pharmaceutically acceptable salts by known methods.

For the preparation of e.g. the methanolate of cefatrizine, the initially obtained reaction mixture is mixed with methanol and subsequently with a dilute aqueous solution of an inorganic acid such as hydrochloride acid, in such a way that finally a pH of 1.0—1.5 of the mixture is attained. From the mixture obtained, the methanolate is prepared in the usual way, such as by adjusting the pH to 1.7—2.4, concentration of the organic phase and addition of an inert organic solvent for a clear separation, extraction of the organic layer with ice water, concentration of the combined water layers, addition of an organic solvent such as ethyl acetate, concentration of the obtained solution, addition of methanol in large excess and adjustment of the pH to 5.5, collecting the precipitate and washing and drying it.

For the preparation of e.g. cefadroxil, this compund is preferably isolated from the initially obtained reaction mixture by mixing it with an aqueous solution of an inorganic acid in such a way that a final pH of abut 1 is attained, purification, addition of N,N-dimethylformamide in large excess, adjustment of pH to 5.5 at about 10°C, collecting of solvate crystals, washing with DMF-water mixtures and drying.

The cefadroxil may be recovered from this solvate by dissolving it in water and addition of seeding crystals of cefadroxil monohydrate.

However, the cefadroxil may also be recovered more directly from the initially obtained reaction mixture by the addition of seeding crystals of cefadroxil-monohydrate to the water phase, obtained after the hydrolysis and purification steps.

A preferred process for the preparation of cefadroxil monohydrate after the cefadroxil is nearly quantitatively isolated from the initially obtained reaction mixture comprises the steps of mixing the cefadroxil with an aqueous solution of an inorganic acid in such a way that a final pH of about 1 is attained, subsequent purification, addition of N,N-dimethylformamide in large excess, adjustment to pH 5.5, collecting the cefadroxil N,N-dimethylformamide solvate crystals, washing them with N,N-dimethylformamide-water mixtures and optionally with other organic solvents, optionally followed by drying, dissolution of the obtained solvate crystals in water and addition of seeding crystals of cefadroxil monohydrate, collection of the crystalline precipitate by filtration, washing and drying and addition of N,N-dimethylformamide in excess to the concentrated mother liquor and washings to recover again the cefadroxil-N,N-dimethylformamide solvate.

It will be appreciated that some of the most important advantages of the acylation process according to the invention are:

—previous and selective silylation of the p-hydroxy group of p-hydroxyphenylglycine is avoided.

—the reaction is carried out in a concentrated solution of the reactants; given the size of the equipment this will favourably influence the output in kilos per batch.

—the use of a p-hydroxyphenylglycine chloro hydrochloride is avoided which can only be prepared by a process which is rather difficult to manage and moreover rather expensive in a number of countries with very stringent safety regulations, if indeed permission for its manufacture can be obtained at all.

—the use of large amounts of other additional chemicals is avoided.

—the desired final product can surprisingly be prepared in economically existing initial yields in a quality acceptable under available health regulations while, at the same time, the number of purification steps can be reduced with attendant smaller losses of desired compound.

—in many cases mixing of solvent is reduced advantageously, so that recovery of solvents used is rather simple and economically favourable, while moreover the starting solvent system can be dried fairly easily.

—the chance of undesired and unexpected crystallization of 7—ACA or its 3-methyl modifications is practically nil, so that a reliable and rather trouble-free process is provided.

It will be appreciated that at least two undesired side reactions always will be considered by skilled people, when acylating by means of a mixed anhydride:

1) acylation, whereby alkoxycarbonylamino cephalosporanic acid derivatives will be formed;

2) partial racemisation of the N-protected amino acid, e.g. during the conversion of the N-protected amino acid or salt thereof into the mixed anhydride.

The avoidance of the first side reaction by rather simple means will be regarded as rather impossible by skilled people relating to the fact that coupling of mixed anhydrides may take place at two reactive sides of the molecule and mixed anhydrides naturally are more or less labile and are inclined to disproportionate into two symmetrical anhydrides. Hence the preparation of the mixed anhydride as well as the conversion of this anhydride with an amino acid will always be carried out at low temperatures.

The second undesired side reaction (racemisation) may be mainly avoided by trial and error methods.

Whereas, according to the literature, ethylchloroformate and — in the event of racemisation when using this reactant, to a somewhat lesser extent — isobutylchloroformate, pivaloylchloride and benzoylchloride were regarded as suitable reactants, the use of methylchloroformate could not be recommended, it now appeared surprisingly that the use of the cheap methylchloroformate leads to significantly improved results.

For the preparation of the mixed anhydride and the subsequent acylation, preferably solvent systems are used, which will meet the requirements of economy (recovery, recycling) and/or ecology. A preferred solvent system for the preparation of e.g. cefadroxil is dichloromethane with a relatively small amount of a suitable co-solvent, such as N-methylpyrrolidone (preferred), tetramethylurea or N,N-dimethylacetamide.

These conditions could surprisingly be fulfilled by the certainly not predictable use of said solvent systems.

The applications of the solvent systems in the preparation of the mixed anhydride surprisingly lead to:

a) an improved and moreover more reproduceable and reliable formation of the mixed anhydride and hence an improved conversion yield to the desired compound;

b) an improved yield of the desired compound in combination with a simultaneous increase of the concentrations to an attractive level, in relation to the present economical requirements;

c) the rather unexpected application of N,O-silylated 7—ACA or derivatives thereof for the reaction with the mixed anhydrides in the indicated yields;

d) the improved conversion yields in the case of the application of co-solvents of the amide type for certain conversions of silylated 7—ACA derivatives;

e) the possibility of carrying out both the sylylation reaction and the preparation of the mixed anhydride in one and the same water-immiscible main solvent, and more particularly dichloromethane, on account of which the presently required recovery of solvents is simplified drastically.

As in industrial processes the application of thoroughly dried solvents and/or of Dane salts of very high purity is an ideal that will never be realized completely, a slight excess of the starting Dane salts and of (lower) alkylchloroformates is preferably used.

The following examples illustrate the process according to the invention, however, without being considered to be restrictive in any respect.

## Example I

Preparation of the monohydrate of 7-[D-α-amino-(p-hydroxyphenyl)acetamido]-3-methyl-3-cephem-4-carboxylic acid.

*a) Preparation of silylated 7-amino-3-methyl-3-cephem-4-carboxylic acid (7—ADCA).*

To 35.0 g (163 mmol) of 7—ADCA were subsequently added 400 ml of dichloromethane and 19.8 g (123 mmol) of hexamethyldisilazane (HMDS). After heating to 38°C, the mixture was refluxed under a dry nitrogen atmosphere for 7.5 hours. The amount of nitrogen, which was passed over per hour, was then about 20 l (of standard conditions). The volume in the reaction flask was maintained on a constant value by the addition of small amounts of dichloromethane as far as necessary. The 7—ADCA was completely dissolved and after the titration of the starting 1N. sulphuric acid solution in a washing bottle connected to the reaction vessel, 98% of the starting HMDS appeared to be taken up as ammonia.

*b) Preparation of methoxycarbonyl D-α-(1-carbomethoxypropen-2-yl) amino-p-hydroxyphenylacetate.*

To 58.0 g of potassium D-α-(1-carbomethoxy-propen-2-yl)-amino-p-hydroxyphenylacetate, 200 ml of dichloromethane were added. After cooling to −40°C, 20 ml of dimethylacetamide were added, while the temperature raised to −33°C. After addition of 0.5 ml of N-methylmorpholine, the mixture was cooled to −38°C and 16.0 ml of methylchloroformate were added in one charge. The temperature raised to about −30°C and the reaction mixture was stirred at this temperature for 2 hours, whereafter the mixture was cooled to −35°C.

*c) Preparation of the dimethylformamidesolvate of 7-[D-α-amino-(p-hydroxyphenyl)acetamido]-3-methyl-3-cephem-4-carboxylic acid (cefadroxil-DMF-solvate).*

The reaction mixture as obtained under a) was cooled to −30°C and added as quickly as possible to the solution of the mixed anhydride as obtained under b) under stirring and cooling, whereby a temperature of −25°C was attained. The reaction mixture was stirred for one hour at −25°C to −20°C. A mixture of 200 ml of distilled water and of 17 ml concentrated hydrochloric acid was added in such a way, that a temperature of 0°C was attained. After 90 minutes stirring at 0°C, hydrolysis appeared to be completed and the pH was 1. The layers were separated and the waterlayer was washed with 100 ml of dichloromethane. The organic layer was washed with 50 ml of distilled water and after extraction of the washing water with the dichloromethane washings, the waterlayer was filtered and the filter was washed with the obtained water washings. Under cooling 1500 ml of dimethylformamide were added and the pH was adjusted to 5.5 at about 10°C by addition of 25% ammonia solution, whereafter crystallization of the cefadroxil-DMF-solvate occurred. After about one hour the crystallization mixture was adjusted to pH 7 and cooled to 0°C. After cooling for one hour at this temperature, the needle-shaped crystals were filtered, washed with 150 ml of a 90% dimethylformamide-water mixture, washed with 300 ml of ethyl acetate and dried *in vacuo* at about 30°C. 73.2 g of a white preparation were obtained. The alleged structure was confirmed by IR and PMR spectra, showing a virtually pure product. Yield 88%.

*d) Preparation of pure crystalline 7-[D-α-amino-p-hydroxyphenylacetamido]-3-methyl-3-cephem-4-carboxylic acid-monohydrate (cefadroxil-monohydrate).*

The DMF-solvate as obtained under c) was added in portions and under stirring in about 10 minutes to 175 ml of distilled water of room temperature. After addition and dissolution of 5 g of the solvate, 1 g of seeding crystals of cefadroxil-monohydrate were added. After the complete addition stirring was continued for one hour. The pyramidial crystals were filtered and washed with water of 0°C and dried under vacuo at about 30°C, giving 35.4 g of a crystalline white product. The alleged structure of the obtained compound was confirmed by IR and PMR spectra, showing a high purity of the obtained product. The mother liquor was evaporated to 50 ml and after addition of 600 ml of DMF, 21.3 g of the same solvate were recovered, giving rise to an overall yield of 78% of cefadroxil, based on the starting 7-ADCA.

Example II

In exactly the same way as in Example I, a-c, a water layer was obtained as under c. This water layer was brought to crystallization without DMF, using 1 g of seeding crystals of the cefadroxil-monohydrate at pH 5. 21.7 g of the desired compound were obtained. After additional treatment of the mother liquor an overall yield of 78.3% could be attained.

Example III

Preparation of silylated 7-amino-3-methyl-3-cephem-4-carboxylic acid.

To 35.0 g of 7-ADCA (163 mmol), 400 ml of dichloromethane and 45.3 ml (327 mmol) of triethylamine were added. Within some minutes 41.5 ml (327 mmol) of trimethylchlorosilane were dropwise added without cooling, while the temperature raised to 38°C. After refluxing for one hour at 38° to 40°C, the reaction mixture was cooled to −30°C. Hereafter 82.2 g of cefadroxil-DMF-solvate were obtained by following the same additional steps b and c of Example I, representing a yield of 98.8%. The alleged structure could be confirmed by IR and PMR spectra, indicating a high purity of the obtained product. According to exactly the same process step d) of Example I, obtained cefadroxil-DMF-solvate was converted into 41.5 g of cefadroxil-monohydrate. The alleged structure of which could be confirmed by IR and PMR spectra, which were also indicating a high purity of the obtained product. According to the Karl Fisher test the moisture content seemed to be 5.5%. From the mother liquor an additional amount of 23 g of DMF-solvate could be obtained, in total representing an overall yield of 89.5% cefadroxil monohydrate, based on the starting 7-ADCA.

**Claims**

1. Process for the preparation of a 7-[D-α-amino-p-hydroxyphenylacetamido] group containing cephalosporanic acid derivative by acylation of a 7-aminocephalosporanic acid derivative or a 3-methyl modification thereof in a water immiscible, inert, organic main solvent with a solution of a mixed anhydride according to the general formula

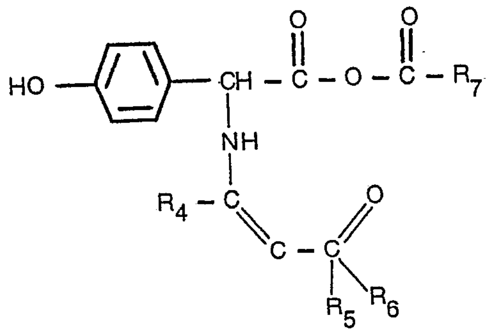

(III)

wherein $R_7$ represents an alkoxy group containing 1—2 carbon atoms, $R_6$ represents an alkoxy group containing 1—3 carbon atoms, $R_5$ represents an alkyl group containing 1—3 carbon atoms or a hydrogen atom, and $R_4$ represents an alkyl group containing 1—3 carbon atoms, which mixed anhydride solution has been previously prepared by reacting the corresponding Dane salt with an alkylchloroformate in an inert, organic main solvent and in the presence of a catalytic amount of a tertiary amine, characterized in that:

a) a 7-aminocephalosporanic acid derivative or a 3-methyl modification thereof is reacted in a dry, inert, organic solvent with a trialkylsilyl group supplying agent in approximately 2 equivalents with respect to 7—ADCA or 7—ACA and approximately 3 equivalents with respect to those 3-methyl modifications which contain a —NH— radical in the residue being attached to the 3-methylene group, whereby the trialkylsilyl group supplying agent in the reaction mixture has been mutually balanced by adjusting at the end of the reaction to an empirically determined signal value as defined in the specification,

b) the obtained reaction mixture, containing a derivative of 7-aminocephalosporanic acid or 3-methyl modification thereof according to the formula

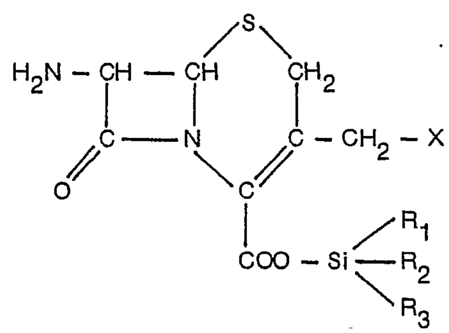

(I)

and/or

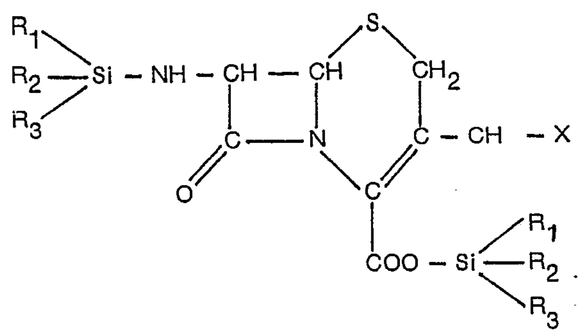

(II)

wherein each of $R_1$, $R_2$ and $R_3$ represents the same or different alkyl group of 1—3 carbon atoms and each preferably represents a methyl group and X represents a residue selected from the group consisting of hydrogen, acetoxy or a five-membered heterocyclic thio ring, containing one or more heteroatoms selected from nitrogen, oxygen and/or sulphur and optionally substituted by alkyl containing 1—3 carbon atoms, is cooled and subsequently is rapidly mixed under anhydrous conditions with a cooled solution of at least an equimolecular amount of a mixed anhydride according to formula (III), which solution has been prepared by reacting the corresponding Dane salt with an alkyl chloroformate under anhydrous conditions in a water immiscible, inert, organic main solvent to which 0—25% by volume of an inert cosolvent has been added and in the presence of a catalytic amount of a tertiary amine, whereafter

c) the acylation reaction is continued at a temperature below 0°C, followed by the recovery of the desired compound by methods known per se.

2. Process according to claim 1, characterised in that the 7-aminocephalosporanic acid derivative or a 3-methyl modification thereof is reacted in dry methylene chloride with trimethylchlorosilane in the presence of a tertiary amine.

3. Process according to claim 2, characterized in that the possibly present amounts of free trimethylchlorosilane and of tertiary amine have mutually been accurately compensated so that the signal recorded by a pH electrode is adjusted at the end of the reaction at a constant value of a "pH scale value" between 5.5 and 7.5 at a temperature between 15—25°C, of a Radiometer pH meter type TTT2, and a Radiometer GK 2401C electrode or an Ingold cold electrode.

4. Process according to claims 1—3, characterized in that the mixed anhydride is previously prepared from the corresponding Dane salt and methyl chloroformate under anhydrous conditions, in the presence of a tertiary amine as catalyst, for example N-methylmorpholine or N,N-dimethylbenzylamine, in dry methylene chloride mixed with a cosolvent or in methyl isobutyl ketone optionally mixed with a cosolvent.

5. Process according to claim 4, characterized in that the mixed anhydride is prepared in dry methylene chloride, to which dimethylformamide, sulfolane, tetrahydrofuran, N-methylpyrrolidone, 1,4-dioxane, acetonitrile, dimethylacetamide or tetramethylurea or a mixture thereof is added as a cosolvent, or in methyl isobutylketone as main solvent to which one or more of the said cosolvents may be added.

6. Process according to claim 5, characterized in that the solvent is dry methylene chloride, to which dimethylformamide, tetrahydrofuran, N-methylpyrrolidone, dimethylacetamide or tetramethylurea or a mixture thereof is added.

7. Process according to claim 6, characterized in that the cosolvent is added in an amount of up to 10% by volume.

8. Process according to any one of claims 1—7, characterized in that the preparation of the Dane mixed anhydride is carried out at a temperature of −10°C to −35°C.

9. Process for preparing the Dane mixed anhydride according to any of claims 1—8, characterized in that sodium or potassium D-α-(1-carbomethoxypropen-2-yl)-amino-p-hydroxyphenylacetate is reacted with methylchloroformate.

10. Process according to any one of the claims 1—9, characterized in that cefadroxil is prepared.

11. Process for the preparation of cefadroxil monohydrate according to claims 1—9, characterized in that the cefadroxil is nearly quantitatively isolated from the initially obtained reaction mixture by mixing it with an aqueous solution of an inorganic acid in such a way that a final pH of about 1 is attained, subsequent purification, addition of N,N-dimethylformamide in large excess, adjustment of pH 5.5, collecting the cefadroxil N,N-dimethylformamide-water mixtures and optionally with other organic solvents, optionally followed by drying, dissolution of the obtained solvate crystals in water and addition of seeding crystals of cefadroxil monohydrate, collection of the crystalline precipitate by filtration, washing and drying and addition of N,N-dimethylformamide in excess to the concentrated mother liquor and washings to recover again the cefadroxil-N,N-dimethylformamide solvate.

12. Process according to any one of the claims 1—9, characterized in that cefatrizine is prepared.

**Patentansprüche**

1. Verfahren zur Herstellung von eine 7-[D-α-Amino-p-hydroxyphenylacetamido]-Gruppe enthaltenden Cephalosporansäure-Derivaten durch Acylierung eines 7-Aminocephalosporansäure-Derivates oder einer 3-Methylmodifizierung davon in einem mit Wasser nicht mischbaren, inerten, organischen Hauptlösungsmittel mit einer Lösung eines gemischten Anhydrides gemäss der allgemeinen Formel:

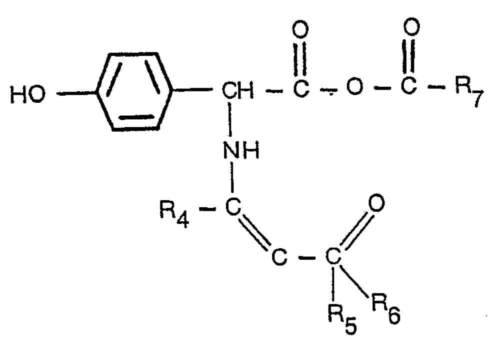

(III)

worin $R_7$ eine 1 oder 2 Kohlenstoffatome enthaltende Alkoxygruppe bedeutet, $R_6$ eine 1 bis 3 Kohlenstoffatome enthaltende Alkoxygruppe bedeutet, $R_5$ eine 1 bis 3 Kohlenstoffatome enthaltende Alkylgruppe oder ein Wasserstoffatom bedeutet und $R_4$ eine 1 bis 3 Kohlenstoffatome enthaltende Alkylgruppe bedeutet, welche gemischte Anhydridlösung vorher hergestellt worden ist durch Umsetzung des entsprechenden Dane-Salzes mit einem Alkylchlorformiat in einem inerten, organischen Hauptlösungsmittel und in Gegenwart einer katalytischen Menge eines tertiären Amins, dadurch gekennzeichnet, dass man

a) ein 7-Aminocephalosporansäure-Derivat oder eine 3-Methyl-modifizierung davon in einem trockenen, inerten, organischen Lösungsmittel mit einem Trialkylsilylgruppen liefernden Mittel in annäherungsweise 2 Aequivalenten in Bezug auf 7-ADCA oder 7-ACA und annäherungsweise 3

Aequivalenten in Bezug auf diejenigen 3-Methylmodifizierungen, die einen —NH-Rest in dem an die 3-Methylengruppe gebundenen Rest enthalten, umsetzt, wobei das Trialkylsilylgruppen liefernde Mittel in dem Reaktionsgemisch gegenseitig ausgeglichen worden ist durch Einstellen am Ende der Reaktion auf einen empirisch bestimmten Signalwert, wie in der Beschreibung definiert,

b) das erhaltene Reaktionsgemisch, das ein Derivat von 7-Aminocephalosporansäure oder einer 3-Methylmodifizierung davon gemäss der Formel:

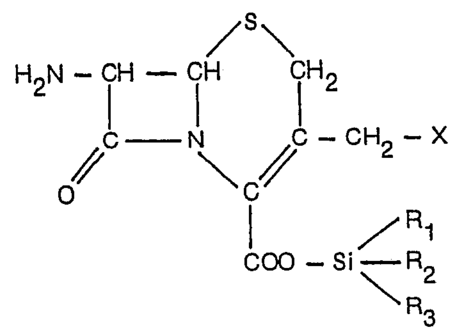

(I)

und/oder

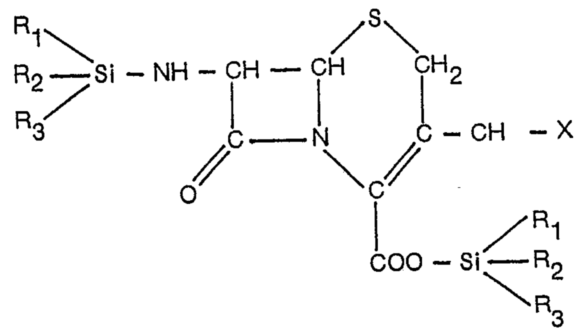

(II)

enthält, worin jedes der Symbole $R_1$, $R_2$ und $R_3$, die die gleiche oder verschiedene Bedeutungen haben, eine Alkylgruppe mit 1 bis 3 Kohlenstoffatomen bedeutet und jedes vorzugsweise eine Methylgruppe bedeutet und X einen Rest bedeutet, der aus der Gruppe gewählt ist, die besteht aus Wasserstoff, Acetoxy oder einem 5-gliedrigen heterocyclischen Thioring, der ein oder mehrere Heteroatome enthält, die aus Stickstoff, Sauerstoff und/oder Schwefel gewählt sind, und der gegebenenfalls durch Alkyl mit 1 bis 3 Kohlenstoffatomen substituiert ist, abkühlt und anschliessend unter wasserfreien Bedingungen schnell mit einer gekühlten Lösung einer mindestens äquimolaren Menge eines gemischten Anhydrides gemäss Formel III mischt, welche Lösung durch Umsetzung des entsprechenden Dane-Salzes mit einem Alkylchlorformiat unter wasserfreien Bedingungen in einem mit Wasser nicht mischbaren, inerten, organischen Hauptlösungsmittel, dem 0 bis 25 Volumen-% eines inerten Colösungsmittels zugesetzt worden sind, und in Gegenwart einer katalytischen Menge eines tertiären Amins hergestellt worden ist, wonach man

c) die Acylierungsreaktion bei einer Temperatur unter 0°C fortsetzt und danach die gewünschte Verbindung mittels an sich bekannter Verfahren gewinnt.

2. Verfahren nach Anspruch 1, dadurch gekennzeichnet, dass man das 7-Aminocephalosporansäure-Derivat oder eine 3-Methylmodifizierung davon in trockenem Methylenchlorid in Gegenwart eines tertiären Amins mit Trimethylchlorsilan umsetzt.

3. Verfahren nach Anspruch 2, dadurch gekennzeichnet, dass die gegebenenfalls vorhandenen Mengen an freiem Trimethylchlorsilan und an tertiärem Amin gegenseitig genau kompensiert worden sind, so dass das durch eine pH-Elektrode registrierte Signal am Ende der Reaktion auf einen konstanten Wert eines "pH-Skalenwertes" eines Radiometer-pH-Meters Typ TTT2 und einer Radiometer GK 2401C-Elektrode oder einer Ingold-kaltelektrode zwischen, 5,5 und 7,5 bei einer Temperatur zwischen 15 und 25°C eingestellt wird.

4. Verfahren nach Anspruchen 1 bis 3, dadurch gekennzeichnet, dass das gemischte Anhydrid vorher unter wasserfreien Bedingungen in Gegenwart eines tertiären Amins als Katalysator, z.B. N-Methylmorpholin oder N,N-Dimethylbenzylamin, in trockenem Methylenchlorid, das mit einem Colösungsmittel gemischt ist, oder in Methylisobutylketon, das gegebenenfalls mit einem Colösungsmittel gemischt ist, aus dem entsprechenden Dane-Salz und Methylchlorformiat hergestellt wird.

5. Verfahren nach Anspruch 4, dadurch gekennzeichnet, dass das gemischte Anhydrid in trockenem Methylenchlorid, zu dem Dimethylformamide, Sulfolan, Tetrahydrofuran, N-Methylpyrrolidon, 1,4-Dioxan, Acetonitril, Dimethylacetamid oder Tetramethylharnstoff oder ein Gemisch davon als Colösungsmittel zugesetzt wird, oder in Methylisobutylketon als Hauptlösungsmittel, zu dem eines oder mehrere der genannten Colösungsmittel zugesetzt werden können, hergestellt wird.

6. Verfahren nach Anspruch 5, dadurch gekennzeichnet, dass das Lösungsmittel trockenes Methylenchlorid ist, zu dem Dimethylformamid, Tetrahydrofuran, N-Methylpyrrolidon, Dimethylacetamid oder Tetramethylharnstoff oder ein Gemisch davon zugesetzt wird.

7. Verfahren nach Anspruch 6, dadurch gekennzeichnet, dass man das Colösungsmittel in einer Menge von bis zu 10 Volumen-% zusetzt.

8. Verfahren nach einem der Ansprüche 1 bis 7, dadurch gekennzeichnet, dass man die Herstellung des Dane-Mischanhydrids bei einer Temperatur von −10 bis −35°C ausführt.

9. Verfahren zur Herstellung des Dane-Mischanhydrids gemäss einem der Ansprüche 1 bis 8, dadurch gekennzeichnet, dass man Natrium- oder Kalium-D-α-(1-carbomethoxypropen-2-yl)-amino-p-hydroxyphenylacetat mit Methylchlorformiat umsetzt.

10. Verfahren nach einem der Ansprüche 1 bis 9, dadurch gekennzeichnet, dass man Cefadroxil herstellt.

11. Verfahren zur Herstellung von Cefadroxil-Monohydrat gemäss Ansprüchen 1 bis 9, dadurch gekennzeichnet, dass man das Cefadroxil aus dem anfänglich erhaltenen Reaktionsgemisch nahezu quantitativ isoliert durch Mischen desselben mit einer wässrigen Lösung einer anorganischen Säure in solcher Weise, dass ein End-pH von ca. 1 erzielt wird, anschliessende Reinigung, Zugabe von N,N-Dimethylformamid in grossem Ueberschuss, Einstellung, auf pH = 5,5, Sammeln der Cefadroxil-N,N-Dimethylformamid-Solvat-Kristalle, Waschen derselben mit N,N-Dimethylformamid-Wasser-Gemischen und gegebenenfalls mit anderen organischen Lösungsmitteln, gegebenenfalls gefolgt von Trocknen, Auflösen der erhaltenen Solvatkristalle in Wasser und Zugabe von Impfkristallen von Cefadroxil-Monohydrat, Sammeln des kristalinen Niederschlages durch Filtration, Waschen und Trocknen und Zugabe von N,N-Dimethylformamid im Ueberschuss zu der konzentrierten Mutterlauge und Waschflüssigkeiten zur erneuten Gewinnung des Cefadroxil-N,N-Dimethylformamid-Solvates.

12. Verfahren nach einem der Ansprüche 1 bis 9, dadurch gekennzeichnet, dass man Cefatrizin herstellt.

**Revendications**

1. Procédé de préparation d'un dérivé d'acide céphalosporanique contenant un radical 7-[D-α-amino-p-hydroxyphényl-acétamido] par acylation d'un dérivé d'acide 7-aminocéphalosporanique ou d'un dérivé modifié 3-méthylé de celui-ci dans un solvant organique principal inerte non miscible à l'eau, au moyen d'une solution d'un anhydride mixte de la formule générale:

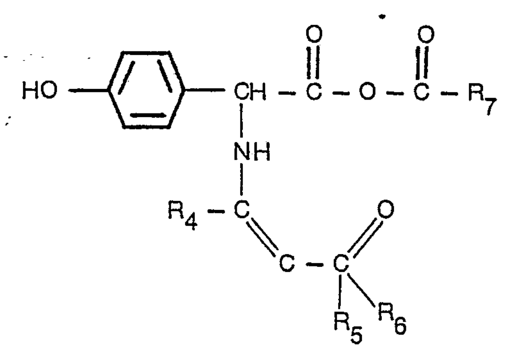

(III)

où $R_7$ représente un radical alcoxy de 1 ou 2 atomes de carbone, $R_6$ représente un radical alcoxy de 1 à 3 atomes de carbone, $R_5$ représente un radical alcoyle de 1 à 3 atomes de carbone, ou un atome d'hydrogène et $R_4$ représente un radical alcoyle de 1 à 3 atomes de carbone, laquelle solution d'anhydride mixte a été préparée au préalable par réaction du sel de Dane correspondant avec un chloroformiate d'alcoyle dans un solvant organique principal inerte et en présente d'une quantité catalytique d'une amine tertiaire, caractérisé en ce que:

a) un dérivé de l'acide 7-aminocéphalosporanique ou un dérivé modifié 3-méthylé de celui-ci est mis à réagir dans un solvant organique inerte sec avec un agent apportant un radical trialcoylsilyle à raison d'environ 2 équivalents par rapport au 7-ADCA ou 7-ACA et d'environ 3 équivalents par rapport aux dérivés modifiés 3-méthylés qui contiennent un radical —NH-dans le résidu uni au radical 3-méthylène, l'agent apportant le radical trialcoylsilyle dans le mélange de réaction ayant été mutuellement équilibré par ajustement, au terme de la réaction, à une valeur de signal déterminée empiriquement telle que définie dans le mémoire;

b) le mélange de réaction obtenu, que contient un dérivé de l'acide 7-aminocéphalosporanique ou un dérivé modifié 3-méthylé de celui-ci de formule:

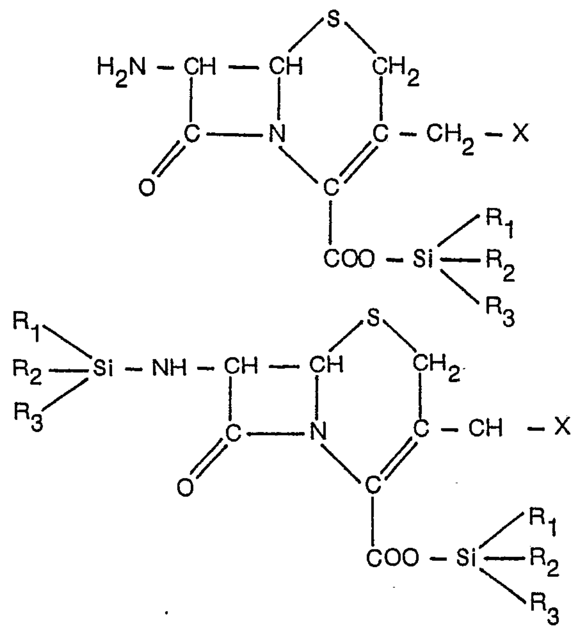

(I)

et/ou

(II)

où chacun d'entre $R_1$, $R_2$ et $R_3$ représente un radical alcoyle identique ou différent de 1 à 3 atomes de carbone et chacun représente, de préférence, un radical méthyle, et X représente un reste choisi dans la classe formée par l'atome d'hydrogène, le radical acétoxy et un thiocycle hétérocyclique à cinq chaînons contenant un ou plusieurs hétéroatomes choisis entre l'azote, l'oxygène et/ou le soufre et éventuellement substitué par un radical alcoyle de 1 à 3 atomes de carbone, est refroidi et est ensuite rapidement mélangé en conditions anhydres avec une solution refroidie d'au moins une quantité équimoléculaire d'un anhydride mixte de formule (III), laquelle solution a été préparée par réaction du sel de Dane correspondant avec un chloroformaite d'alcoyle en conditions anhydres dans un solvant organique principal inerte non miscible à l'eau auguel un co-solvant inerte a été ajouté à raisòn de 0 à 25% en volume et en présence d'une quantité catalytique d'une amine tertiaire, apre6s quio

c) la réaction d'acylation est poursuive à une température inférieure à 0°C et est suivie de l'isolement du composé souhaité suivant les technniques connues.

2. Procédé suivant la revendication 1, caractérisé en ce que le dérivé d'acide 7-aminocéphalosporanique ou un dérivé modifié 3-méthylé de celui-ci est mis à réagir dans le chlorure de méthylène sec avec le triméthylchlorosilane en présence d'une amine tertiaire.

3. Procédé suivant la revendication 2, caractérisé en ce que les quantités éventuellement en présence de triméthylchlorosilane libre et d'amine tertiaire ont été mutuellement compensées avec précision, de façon que le signal enregistré par une électrode de pH soit ajusté au terme de la réaction à une valeur constante d'une "valeur d'échelle de pH" entre 5,5 et 7,5 à une température entre 12 et 25°C, d'un pH-mètre Radiometer type TTT2, et d'une électrode Radiometer GK 2401C ou d'une électrode Ingold froide.

4. Procédé suivant les revendications 1—3, caractérisé en ce que l'anhydride mixte est préparé au préalable à partir du sel de Dane correspondant et de chloroformiate de méthyle dans des conditions anhydres en présence d'une amine tertiaire comme catalyseur, par exemple la N-méthylmorpholine ou la N,N-diméthylbenzylamine dans du chlorure de méthylène sec mélangé avec un co-solvant ou dans la méthylisobutylcétone éventuellement mélangée avec un co-solvant.

5. Procédé suivant la revendication 4, caractérisé en ce que l'anhydride mixte est préparé dans le chlorure de méthylène sec auquel du diméthylformamide, du solfolane, du tétrahydrofuranne, de la N-méthyl-pyrrolidone, du 1,4-dioxanne, de l'acétonitrile, du diméthylacétamide ou de la tétraméthylurée ou bien un mélange de ceux-ci est ajouté comme co-solvant ou dans de la méthylisobutylcétone comme solvant principal à laquelle un ou plusieurs de ces co-solvants jpeuvent être ajouté.

6. Procédé suivant la revendication 5, caractérisé en ce que le solvant est le chlorure de méthylène sec auquel du diméthylformamide, du tétrahydrofuranne, de la N-méthyl-pyrrolidone, du diméthylacétamide ou de la tétraméthylurée ou un mélange de ceux-ci est ajouté.

7. Procédé suivant la revendication 6, caractérisé en ce que le co-solvant est ajouté en une quantité s'élevant jusqu'à 10% en volume.

8. Procédé suivant l'une quelconque des revendications 1 à 7, caractérisé en ce que la préparation de l'anhydride mixte de Dane est exécutée à une température de −10°C à −35°C.

9. Procédé de préparation de l'anhydride mixte de Dane suivant l'une quelconque des revendications 1 à 8, caractérisé en ce que le D-α-(1-carbométhoxy-propène-2-yl)-amino-p-hydroxyphényle-acétate de sodium ou de potassium est mis à réagir avec le chloroformiate de méthyle.

10. Procédé suivant l'une quelconque des revendications 1 à 9, caractérisé en ce que le céfadroxil est préparé.

11. Procédé de préparation du céfadroxil monohydraté suivant les revendications 1 à 9, caractérisé en ce que le céfadroxil est isolé presque quantitativement du mélange de réaction obtenu initialement par mélange de celui-ci avec une solution aqueuse d'un acide inorganique de façon qu'un pH final d'environ 1 soit atteint, puis purification, addition de N,N-diméthylformamide en grand excès, ajustement du pH à 5,5, collecte des cristaux du solvate N,N-diméthylformamidique de céfadroxil, lavage de ceux-ci avec des mélanges, N,N-diméthylformamide-eau et éventuellement avec d'autres solvants organiques, éventuellement ensuite séchage, dissolution des cristaux de solvate résultants dans de l'eau et addition de germes cristallins de cefadroxil monohydraté, collecte du précipité cristallin par filtration, lavage et séchage et addition de N,N-diméthylformamide en excès à la liqueur mère concentrée et aux liqueurs de lavage pour isoler à nouveau le solvate N,N-diméthylformamidique de céfadroxil.

12. Procédé suivant l'une quelconque des revendications 1 à 9, caractérisé en ce que la céfatrizine est préparée.